# EUROPEAN PATENT SPECIFICATION

(11) **EP 1 743 606 B1**
(45) Date of publication and mention of the grant of the patent: **11.03.2009**
(21) Application number: 06253714.7
(22) Date of filing: 14.07.2006
(51) Int. Cl.: A61F 5/00

(54) **Accordion-like gastric band**
Akkordeonförmiges Magenband
Bande gastrique de type accordéon

(30) Priority: 15.07.2005 US 182071
(43) Date of publication of application: 17.01.2007
(73) Proprietor: ETHICON ENDO-SURGERY, INC., Cincinnati, Ohio 45242 (US)
(72) Inventor: Tsonton, Mark, Loveland OH 45140 (US)
(74) Representative: Tunstall, Christopher Stephen

(56) References cited:
- EP-A1- 1 342 458
- US-A- 4 592 339

## Description

### BACKGROUND OF THE INVENTION

### 1. Field of the Invention

The invention relates to a gastric band. More particularly, the invention relates to a balloon-based gastric band having an "accordion-like" construction allowing for improved expansion and contraction. The closest prior art is EP-A-1 342 458, which defines the preamble of claim 1.

### 2. Description of the Prior Art

Morbid obesity is a serious medical condition. In fact, morbid obesity has become highly pervasive in the United States, as well as other countries, and the trend appears to be heading in a negative direction. Complications associated with morbid obesity include hypertension, diabetes, coronary artery disease, stroke, congestive heart failure, multiple orthopedic problems and pulmonary insufficiency with markedly decreased life expectancy. With this in mind, and as those skilled in the art will certainly appreciate, the monetary and physical costs associated with morbid obesity are substantial. In fact, it is estimated the costs relating to obesity are in excess of 100 billion dollars in the United States alone.

A variety of surgical procedures have been developed to treat obesity. The most common currently performed procedure is Roux-en-Y gastric bypass (RYGB). This procedure is highly complex and is commonly utilized to treat people exhibiting morbid obesity. Other forms of bariatric surgery include Fobi pouch, bilio-pancreatic diversion, and gastroplastic or "stomach stapling". In addition, implantable devices are known which limit the passage of food through the stomach and affect satiety.

In view of the highly invasive nature of many of these procedures, efforts have been made to develop less traumatic and less invasive procedures. Gastric-banding is a type of gastric reduction surgery attempting to limit food intake by reducing the size of the stomach. In contrast to RYGB and other stomach reduction procedures, gastric banding does not require the alteration of the anatomy of the digestive tract in the duodenum or jejunum.

Since the early 1980s, gastric bands have provided an effective alternative to gastric bypass and other irreversible surgical weight loss treatments for the morbidly obese. Several alternative procedures are performed under the heading of gastric-banding. Some banding techniques employ a gastric ring, others use a band, some use stomach staples and still other procedures use a combination of rings, bands and staples. Among the procedures most commonly performed are lap band, vertical banded gastroplasty (VBG), silastic ring gastroplasty (SRG), and adjustable silastic gastric banding (AGB).

In general, the gastric band is wrapped around an upper portion of the patient's stomach, forming a stoma that is less than the normal interior diameter of the stomach that restricts food passing from an upper portion to a lower digestive portion of the stomach. When the stoma is of an appropriate size, food held in the upper portion of the stomach provides a feeling of fullness that discourages overeating.

Typically, the gastric band is introduced into a patient's abdomen by pushing it through a trocar. As such, it is desirable to reduce the diameter of the band to aid in transport through a trocar.

Gastric bands with balloon constructions have also been developed. Kuzmak et al., in U.S. Pat. No. 4,592,339, teaches a stoma-adjustable gastric band that includes a balloon section that is expandable and deflatable through a remote injection site. The balloon expandable section adjusts the size of the stoma opening both intraoperatively and post-operatively.

During the last several years, manufacturers of prior art gastric bands have improved the designs of the balloons of these bands. One significant area of further improvement, however, is the development of a gastric band that includes a balloon which overcomes problems associated with expansion and contraction while being inserted through a trocar. The present invention, therefore, provides such a gastric band.

### SUMMARY OF THE INVENTION

It is, therefore, an object of the present invention, which is defined in claim 1, to provide a gastric band including a balloon shaped and dimensioned to circumscribe the stomach at a predetermined location. The balloon includes a longitudinally extending body with at least one crease formed therein. The crease extends along a longitudinal axis of the body for providing the balloon with a reduced noninflated profile without reducing the fill volume of the fully expanded balloon.

It is also an object of the present invention to provide a gastric band including fluid supply tube fluidly communicating with the balloon for controlled inflation thereof.

It is another object of the present invention to provide a gastric band including a fluid injection port in fluid communication with the fluid supply tube.

It is a further object of the present invention to provide a gastric band wherein the balloon is composed of silicone.

It is also another object of the present invention to provide a gastric band including a belt secured to the balloon, wherein the belt is shaped and dimensioned to circumscribe the stomach at a predetermined location.

It is still another object of the present invention to provide a gastric band including a belt secured to the balloon, wherein the belt is composed of silicone.

It is yet a further object of the present invention to provide a gastric band wherein the balloon is affixed to an inner surface of the belt.

It is also an object of the present invention to provide a gastric band wherein the belt is intregrally formed with the balloon.

It is another object of the present invention to provide a gastric band wherein the balloon includes a single cavity.

It is a further object of the present invention to provide a gastric band including a fastening mechanism for selectively securing the gastric band in an encircled position around a portion of the stomach.

Other objects and advantages of the present invention will become apparent from the following detailed description when viewed in conjunction with the accompanying drawings, which set forth certain embodiments of the invention.

### BRIEF DESCRIPTION OF THE DRAWINGS

Figure 1 is a perspective view of the present gastric band wrapped about a stomach.
Figure 2 is a perspective view of the preset gastric band.
Figure 3 is a top view of the present gastric band in its circular configuration.
Figure 4 is a cross sectional view along the line 4-4 in Figure 3.

### DESCRIPTION OF THE PREFERRED EMBODIMENT

The detailed embodiment of the present invention is disclosed herein. It should be understood, however, that the disclosed embodiment is merely exemplary of the invention, which may be embodied in various forms. Therefore, the details disclosed herein are not to be interpreted as limiting, but merely as the basis for the claims and as a basis for teaching one skilled in the art how to make and/or use the invention.

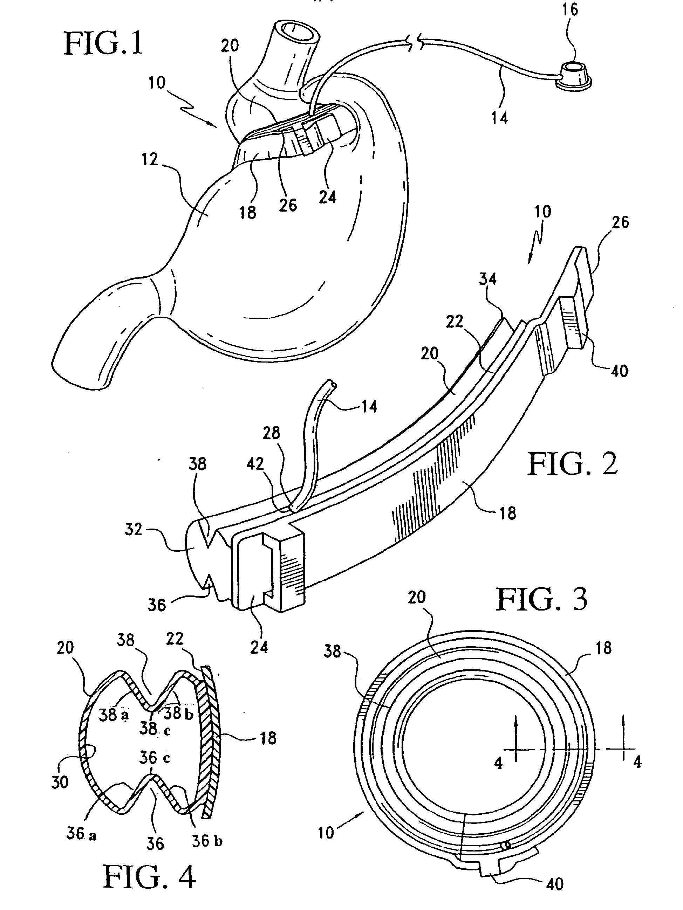

With reference to Figures 1 to 4, a balloon based gastric band 10 in accordance with the present invention is disclosed. The gastric band 10 is shaped and dimensioned to circumscribe the stomach 12 at a predetermined location reducing the size of the stomach.

A fluid supply tube 14 fluidly communicating with gastric band 10 is in fluid communication with a remotely located fluid injection port 16. The gastric band 10 generally comprises an outer tension carrying belt 18 having an inflatable balloon 20 affixed to the inner surface 22 thereof. When the gastric band 10 is in place, the balloon 20 is inflated, restricting the volume of stomach. To inflate the balloon 20, a filling solution is injected into the injection port 16 and the filling solution is conveyed to the balloon 20 by way of the supply tube 14.

Referring to Figure 1, the gastric band 10 is shown wrapped around an upper portion of a stomach 12. The gastric band 10 is kept in place by attaching the first and second ends 24, 26 of the band 10 together and extending a portion of the stomach 12 over the gastric band 10 by suturing a portion to the stomach 12.

Referring now to Figures 2 to 4, the gastric band 10 in accordance with the present invention is disclosed in greater detail. As briefly mentioned above, the gastric band 10 includes a tension carrying belt 18, a fluid supply tube 14 in fluid communication with a balloon 20 and a remotely located fluid injection port 16. The inflatable balloon 20 is carried along the inner surface 22 of the belt 18. The balloon 20 is secured to the inner surface 22 of the belt 18 in any well known manner, or even made of unitary construction with the belt 18, although the belt 18 is typically formed separately.

A distal end 28 of the fluid supply tube 14 is in fluid communication with the internal cavity 30 of the balloon 20 and the proximal end 27 is in fluid communication with an internal cavity (not shown) of the remote injection port 16.

The balloon 20 is an elongated structure with a first end 32 and a second end 34. The balloon 20 is preferably of a length of about 11 cm. However, those skilled in the art will appreciate the balloon 20 may be of any length which would provide sufficient compression of the stomach 12. The balloon 20 is preferably comprised of material with a thickness between about 0.3 mm and 0.7 mm and more preferably about 0.64 mm. The thickness of the material is dependent on the balloon material and it should be appreciated the thickness of the balloon may vary depending on the balloon composition. In accordance with a preferred embodiment of the present invention, the balloon 20 is manufactured from medical grade silicone, although other known materials, for example, implantable polyurethane, may be used without departing from the scope of the present invention.

In accordance with a preferred embodiment of the present invention, the balloon 20 is an elongated body with a single cavity. However, various balloon designs could be used within the scope of the present invention. For example, a multiple segmented balloon as disclosed in U.S. Patent Application Publication No. 2005/0070937, entitled "SEGMENTED GASTRIC BAND", could be employed without departing from the scope of the present invention.

As best shown in Figures 2 to 4, the balloon 20 is formed with first and second creased sections 36, 38. The first and second creased sections 36, 38 are formed at diametrically opposed locations along the circumference of the balloon 20. As will be discussed below in greater detail, the creased sections 36, 38 allow for a reduction in the size of the balloon 20 in a manner improving the insertion of the balloon 20 through a trocar.

The creased sections 36, 38 extend along the length of the balloon 20 and, thereby, allow for the compression of the balloon 20 to a low profile configuration during insertion without adjusting the fill volume of the fully expanded balloon 20. The creased sections 36, 38 provide a controlled compression point along the length of the balloon 20, allowing the balloon 20 to be compressed in a controlled and reliable manner along the length of the balloon. Each of the creased sections 36, 38 is composed of opposed walls 36a, 36b, 38a, 38b linked by a hinged section 36c, 38c. In this way, the creased sections 36, 38 open and close in the same manner as an accordion.

By providing for the increased compression of the balloon 20 in a controlled manner, potential damage to the balloon 20 is decreased during the gastro-gastric suturing step of the application procedure. In addition, the lower profile design allows for use of the present gastric band with small diameter trocars (for example, 12 mm), which previously had difficulty in delivering such balloon, based gastric bands in a reliable and controlled manner.

As those skilled in the art will certainly appreciate, the creases may be formed in a variety of configurations without departing from the scope of the present invention. For example, more than 2 creases could be employed and the creases could be configured to overlap in a mating configuration, such as, with pleats on pants.

The tension-carrying belt 18 is slightly longer than the balloon 20 and may be of any suitable length sufficient to accommodate the type of latching mechanism 40 employed. As with the balloon 20 itself, the carrying belt 18 is composed of medical grade silicone polymer but may be composed of any flexible biocompatible material, for example, implantable polyurethane, without departing from the scope of the present invention.

The gastric band 10, and more particularly, the tension carrying belt 18, is provided with a latching mechanism 40 so that the gastric band 10 may be releasably secured in an encircled position around a portion of the stomach 12. Various latching mechanism may be used within the scope of the present invention. Some of these latching mechanisms are disclosed in U.S. Published Patent Application Nos. 2005/0002984, entitled "IMPLANTABLE BAND WITH ATTACHMENT MECHANISM HAVING DISSIMALR MATERIAL PRORPERTIES", 2004/0267291, entitled "IMPLANTABLE BAND WITH NON-MECHANICAL ATTACHMENT MECHANISM", 2004/0267292, entitled "IMPLANTABLE BAND WITH TRANSVERSE ATTACHMENT MECHANISM", 2004/0267288, entitled "IMPLANTABLE BAND HAVING IMPROVED ATTACHMENT MECHANISM", and 2004/0267293, entitled "IMPLANTABLE BAND WITH ATTACHMENT MECHANISM".

As mentioned above, the balloon 20 is provided with a fluid supply tube 14 coupled to a remote injection port 16. The fluid supply tube 14 includes inlets 42 providing access to the internal cavity 30 of the balloon 20. The remote injection port 16 includes a silicone septum. At the time the adjustable gastric band 10 is implanted around a portion of the stomach 12, the remote injection port 16 is also implanted at a suitable location, usually within the rectus sheaths, for transcutaneous access via a Huber needle. Examples of injection port structures which may employed in accordance with the present invention are disclosed in U.S. Patent Application Publication Nos. 2004/0254536, entitled "SUBCUTANEOUS SELF ATTACHING INJECTION PORT WITH INTEGRAL FASTENERS", and 2004/0254537, entitled "SUBCUTANEOUS SELF ATTACHING INJECTION PORT WITH INTEGRAL MOVEABLE RETENTION MEMBERS".

The internal cavity 30 of the balloon 20 is evacuated prior to installation. The fluid supply tube 14 and the internal cavity of the remote injection port 16 are preferably supplied with physiologically compatible fluids, such as a saline or radiopaque solutions, during postoperative adjustment. Postoperative adjustment of the perimeter enclosed by the balloon 20, and therefore the size of the stoma, is accomplished by the addition or removal of fluid from the internal cavity 30 of the balloon 20 by inserting a Huber needle percutaneously into the silicone septum of the injection port 16.

Installation of the gastric band 10 is accomplished by first inserting the band 10 into the patient's abdomen through a trocar. Next, a tunnel is created behind the stomach 12 near the esophagogastric junction using a blunt dissection device. The gastric band 10 is then grasped by an instrument, such as a grasper or blunt dissection device, and wrapped around the patient's stomach 12 through the created tunnel. The latching mechanism 40 is then engaged. The injection port 16 is then attached to the gastric band 10 and the injection port 16 is secured subcutaneously in the abdomen or other suitable location. A suitable filling solution, such as saline, is then injected into injection port 16 whereby the solution is conveyed to the internal cavity 30 of the balloon by way of inlets 42 in fluid supply tube 14. If necessary either at the time the gastric band 10 is installed or at some time in the future, a predetermined quantity of the filling solution may be withdrawn for the balloon 20 by inserting a syringe into the injection port 16 and withdrawing the solution.

Although the present invention is described for use in conjunction with gastric bands, those skilled in the art will appreciate the above invention has equally applicability to other types of implantable bands. For example, bands are used for the treatment of fecal incontinence. One such band is described in U.S. Pat. No. 6,461,292. Bands can also be used to treat urinary incontinence. One such band is described in U.S. Patent Application Publication No. 2003/0105385. Bands can also be used to treat heartburn and/or acid reflux. One such band is described in U.S. Patent No. 6,470,892. Bands can also be used to treat impotence. One such band is described in U.S. Patent Application Publication No. 2003/0114729.

While the preferred embodiments have been shown and described, it will be understood that there is no intent to limit the invention by such disclosure, but rather, is intended to cover all modifications and alternate constructions falling within the scope of the invention.

## Claims

1. A gastric band (10), comprising:
a balloon (20) shaped and dimensioned to circumscribe the stomach at a predetermined location;
the balloon having a first end (32) and a second end (34) and including a longitudinally extending body with at least one crease (36, 38) formed therein, the crease extending along a longitudinal axis of the body for providing the balloon with a reduced noninflared profile without reducing the fill volume of the fully expanded balloon, and **characterized by**;
the crease extending along the length of the balloon;
the first end and the second end of the balloon being adapted to be in abutting contact with one another.

2. The gastric band according to claim 1, further including fluid supply tube (14) fluidly communicating with the balloon for controlled inflation thereof.

3. The gastric band according to claim 2, further including a fluid injection port (16) in fluid communication with the fluid supply tube.

4. The gastric band according to claim 1, wherein the balloon is composed of silicone.

5. The gastric band according to claim 1, further including a belt secured to the balloon, wherein the belt is shaped and dimensioned to circumscribe the stomach at a predetermined location.

6. The gastric band according to claim 1, further including a belt secured to the balloon, wherein the belt is composed of silicone.

7. The gastric band according to claim 5, wherein the balloon is affixed to an inner (22) surface of the belt.

8. The gastric band according to claim 5, wherein the belt (18) is intregrally formed with the balloon.

9. The gastric band according to claim 1, wherein the balloon includes a single cavity.

10. The gastric band according to claim 1, further including a fastening mechanism for selectively securing the gastric band in an encircled position around a portion of the stomach.

## Patentansprüche

1. Magenband (10), das aufweist:
einen Ballon (20), der so geformt und bemessen ist, dass er den Magen an einem vorbestimmten Ort umgibt;
wobei der Ballon ein erstes Ende (32) und ein zweites Ende (34) hat und einen sich in Längsrichtung erstreckenden Körper umfasst, in dem wenigstens eine Falte (36, 38) ausgebildet ist, wobei sich die Falte entlang einer Längsachse des Körpers erstreckt, um den Ballon mit einem verringerten, nicht aufgeweiteten Profil zu versehen, ohne das Füllvolumen des vollständig aufgeweiteten Ballons zu verringern, und **dadurch gekennzeichnet, dass:**
die Falte sich über die Länge des Ballons erstreckt;
das erste Ende und das zweite Ende des Ballons so angepasst sind, dass sie aneinander anliegen.

2. Magenband nach Anspruch 1, das weiter eine Fluidzufuhrleitung (14) aufweist, das für dessen gesteuerte Aufweitung in fluidischer Verbindung mit dem Ballon steht.

3. Magenband nach Anspruch 2, das weiter einen Fluideinlassport (16) in fluidischer Verbindung mit der Fluidzufuhrleitung umfasst.

4. Magenband nach Anspruch 1, bei dem der Ballon aus Silikon aufgebaut ist.

5. Magenband nach Anspruch 1, das weiter einen Gurt umfasst, der an dem Ballon gesichert ist, wobei der Gurt so geformt und bemessen ist, dass er den Magen an einem vorbestimmten Ort umgibt.

6. Magenband nach Anspruch 1, das weiter einen Gurt umfasst, der an dem Ballon gesichert ist, wobei der Gurt aus Silikon aufgebaut ist.

7. Magenband nach Anspruch 5, bei dem der Ballon an einer inneren (22) Fläche des Gurtes befestigt ist.

8. Magenband nach Anspruch 5, bei dem der Gurt (18) einheitlich mit dem Ballon ausgebildet ist.

9. Magenband nach Anspruch 1, bei dem der Ballon einen einzigen Hohlraum umfasst.

10. Magenband nach Anspruch 1, das weiter einen Befestigungsmechanismus zum ausgewählten Sichern des Magenbandes in einer einschließenden Lage um einen Bereich des Magens umfasst.

## Revendications

1. Bande gastrique (10), comprenant :
■ un ballonnet (20) formé et dimensionné pour restreindre l'estomac à un emplacement prédéterminé ;
■ le ballonnet ayant une première extrémité (32) et une seconde extrémité (34) et comprenant un corps s'étendant de manière longitudinale avec au moins un pli (36, 38) formé à l'intérieur de celui-ci, le pli s'étendant le long d'un axe longitudinal du corps pour doter le ballonnet d'un profil réduit non gonflé sans réduire le volume de remplissage du ballonnet complètement expansé, et **caractérisée par** :
■ le pli qui s'étend le long de la longueur du ballonnet ;
■ la première extrémité et la seconde extrémité du ballonnet qui sont adaptées pour être en contact de butée l'une par rapport à l'autre.

2. Bande gastrique selon la revendication 1, comprenant en outre un tube d'alimentation de fluide (14) qui communique de manière fluidique avec le ballonnet pour son gonflage contrôlé.

3. Bande gastrique selon la revendication 2, comprenant en outre un orifice d'injection de fluide (16) en communication de fluide avec le tube d'alimentation de fluide.

4. Bande gastrique selon la revendication 1, dans laquelle le ballonnet est composé de silicone.

5. Bande gastrique selon la revendication 1, comprenant en outre une ceinture fixée au ballonnet, dans laquelle la ceinture est formée et dimensionnée pour restreindre l'estomac à un emplacement prédéterminé.

6. Bande gastrique selon la revendication 1, comprenant en outre une ceinture fixée sur le ballonnet, dans laquelle la ceinture est composée de silicone.

7. Bande gastrique selon la revendication 5, dans laquelle le ballonnet est fixé à une surface interne (22) de la ceinture.

8. Bande gastrique selon la revendication 5, dans laquelle la ceinture (18) est formée de manière solidaire avec le ballonnet.

9. Bande gastrique selon la revendication 1, dans laquelle le ballonnet comprend une seule cavité.

10. Bande gastrique selon la revendication 1, comprenant en outre un mécanisme de fixation pour fixer de manière sélective la bande gastrique dans une position encerclée autour d'une partie de l'estomac.
